(19) Europäisches Patentamt
European Patent Office
Office européen des brevets

(11) Publication number: 0 252 558 A2

# (12) EUROPEAN PATENT APPLICATION

(21) Application number: 87201239.8

(22) Date of filing: 29.06.87

(51) Int. Cl.4: C12N 15/00 , C12N 1/20 , C12Q 1/68

The microorganism(s) has (have) been deposited with ATCC under number(s) 67011.

(30) Priority: 04.07.86 IT 2103186

(43) Date of publication of application: 13.01.88 Bulletin 88/02

(84) Designated Contracting States: AT BE CH DE ES FR GB GR LI LU NL SE

(71) Applicant: SCLAVO S.p.A.
Via Fiorentina 1
I-53100 Siena(IT)

(72) Inventor: Rappuoli, Rino
Via Calamandrei 35
I-Quercegrossa-Monteriggioni Siena(IT)
Inventor: Perugini, Maria
Via Colombini 3
I-53100 Siena(IT)
Inventor: Ratti, Giulio
Via Strada di Vico Alto 6
I-53100 Siena(IT)

(74) Representative: Roggero, Sergio et al
Ing. Barzanò & Zanardo Milano S.p.A. Via Borgonuovo 10
I-20121 Milano(IT)

## (54) Element of dna of corynebacterium diphteriae with typical properties of an is insertion element.

(57) An element of DNA of about 1,500 base-pairs is disclosed, which is endowed with the typical properties of an IS insertion element, present in one or more copies, and on different sites in the chromosomal DNA of strains of C. diphtheriae.

Molecule of recombinant DNA obtained from the splicing of a clonation vector with a DNA segment containing said insertion element or fragments thereof, and microorganism transformed with said molecule of recombinant DNA.

The IS insertion element is useful for the preparation of strain-specific hybridization probes, which can be used for the characterization and the epidemiological study of strains of C. diphtheriae.

Fig.2

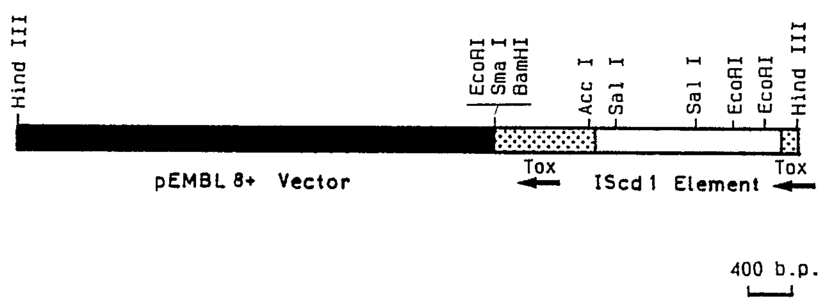


EP 0 252 558 A2

"ELEMENT OF DNA OF CORYNEBACTERIUM DIPHTHERIAE WITH TYPICAL PROPERTIES OF AN IS INSERTION ELEMENT"

The present invention relates to an element of DNA endowed with typical properties of an IS insertion element, present in one or more copies and on different sites of the chromosomal DNA of strains of Corynebacterium diphtheriae (C. diphtheriae) useful for the preparation of strain-specific hybridization probes.

The invention relates furthermore to a molecule of recombinant DNA obtained from the union of a cloning segment with a DNA segment containing said insertion element or fragments thereof, and to a microorganism transformed by means of said molecule of recombinant DNA.

The present invention relates also to the use of said hybridization probes for the characterization and the epidemiological study of strains of C. diphtheriae.

The diphtheria is a disease which strikes man, and, in particular, the children in their infantile age, and manifests itself with local lesions of the mucosae, consisting is an inflammatory process with fibrinous exudate and necrosis.

Its clinical course is accompanied by general signs of intoxication such as fever and anemia and, in the most serious cases, by delirium, convulsions and circulatory and renal disorders.

Furthermore, on occurred healing, complications of paralytic type and myocarditis may result, which, in some cases, may lead to a sudden death due to cardiacal paralysis.

The etiologic agent of diphtheria is Corynebacterium diphtheriae, a gram-positive bacterium which, after lysogenization with a temperate phage containing the toxin-codifying gene (tox), acquires the capability of producing the diphtherial toxin.

Among the strains of C. diphtheriae, three types can be distinguished, on the basis of a different level of pathogenicity, i.e., the mitis type, the gravis type and the intermedius type and, within the same type strains are present which, even if show a different toxinogenicity, cannot be easily morphologically distinguished on the basis of their bacteriophage.

Presently, the diagnostic ascertainment of diphtheria is carried out by means of conventional techniques, such as, e.g., the culture of the sample under examination on suitable media and toxigenic tests.

The cultivation methods are however very laborious, they require, in fact, long analysis times, and the need of keeping alive the bacteria present in the sample during their transport and laboratory examination.

Furthermore, by means of said techniques, it is not always possible to distinguish C. diphtheriae from such pseudodiphtherial bacteria as C. hoffmani and C. xerosis, present with a relative frequency in the pharyngeal mucus and in the saliva of healthy individuals, and with morphologic and cultivation characteristics very similar to those of C. diphtheriae.

Summing up, these classic methods show many disadvantages such as long work times and poor specificity.

It derives therefrom the need of having available alternative diagnostic techniques which allow the drawbacks of the prior art to be overcome.

Among these, the nucleotidic hybridization technique can be, e.g., mentioned, which allows the presence of pathogens in a sample under examination to be detected with a high efficiency and specificity, by using fragments of DNA or of RNA, the nuclotidic sequence of which is complementary of or homologous to a sequence of the bacterial genome.

The use of the nucleotidic hybridization is essentially based on the double-spiral structure of DNA and on the mutual complementarity between the nucleotidic bases. By operating according to said technique, the hydrogen bond between the bases of the individual DNA spirals can be split under suitable conditions and one of the individual spirals resulting from this denaturation, when contacted with an individual, suitably labeled DNA or RNA chain, and said hybridization probe, which contains a sequence of bases sufficiently complementary or homologous to the first one, generates, under suitable reaction conditions, such hybrids as DNA/DNA, DNA/RNA or RNA/RNA.

In as much as said hybridization technique determines the DNA sequence, it derives therefrom that the sensitivity and the specificity of the method can be improved if a probe is available which is capable of recognizing a repetitive DNA sequence.

Furthermore, if this repetitive sequence is species-specific of strain-specific, also the probe capable of recognizing said sequence will be species-specific or strain-specific.

Generally, a DNA or RNA probe is selected on the basis of the fact that it comprises a sequence of bases essentially present in all interesting bacteria.

The sequences of homologous bases useful for the preparation of the hybridization probes can be selected from genes or fragments thereof, the stability of which in the bacteria is known, or is determined by experimental methods.

The selected genes can be structural genes, regulatory genes or genes performing different functions.

Furthermore, the probes can be selected from genes which codify for membrane proteins, genes which codify for the insertion sequences, transposons and the like.

In particular, the transposable elements are DNA sequences constituted by a number of base-pairs variable from 500 to 10,000 bases, which can shift and insert themselves in different points of the bacterial genome, giving rise to that process which is called "transposition".

The transposition can cause the activation or the deactivation of genes of the host cell, and constitutes a mechanism of production of genetic variability.

These elements indeed, when they insert themselves in the nearby of, or inside, a gene, inhibit its function, whilst, when they leave their insertion site, they allow the gene to restart its function, with the restoration of the original phenotype.

The transposable elements of the bacteria are subdivided into three classes:

-simple transposons, or simple IS elements;

-composed transposons or TN; and

-complex transposons.

In particular, the IS elements, the insertion sequences of which are normal constituents of the bacterial genome, have dimensions ranging from 800 to 2000 base-pairs, and bear, at each of their ends, a short repeated and inverted base sequence.

Furthermore, on the insertion site, the IS is flanked by two short equal and direct (not inverted) sequences generally consisting of from 5 to 9 base-pairs, and the length of which remains constant for a determined transposon.

In the prokaryotes the IS and TN elements are ubiquitary and have been found in the chromosomes, in the plasmids and in bacteriophages. However, the most of the IS and TN elements described in the technical literature have been isolated from E. coli or other gram-negative bacteria, and very little is known about the presence of these elements in the gram-positive bacteria, and, in particular, in C. diphtheriae.

The unavailability of said elements for C. diphtheriae did not allow its genome, very little known, to be studied.

A DNA element was found now, which is endowed with the typical characteristics of an IS insertion element, present in one or more copies and on different sites in the chromosomal DNA of strains of C. diphtheriae, which allows the problems of the prior art to be overcome.

The present invention is based on the observation that the DNA segment, inserted in the structural gene of the toxin of diphtheria, and responsible for $tox^-$ phenotype of gamma bacteriophage, shows typical characteristics of an IS insertion element, and that said element is present, in one or more copies and on different sites in the chromosomal DNA of strains of C. diphtheriae is a way stable and typical for the various strains.

In accordance with the above, a purpose of the present invention is a DNA element of about 1,500 base-pairs, with typical properties of an IS insertion element, present in one or more copies, and on different sites in the chromosomal DNA of strains of C. diphtheriae, useful for the preparation of strain-specific hydridization probes.

Another purpose of the present invention is a molecule of recombinant DNA obtained from the union of a cloning vector with a DNA segment containing said IS element, or fragments thereof, and a microorganism transformed with said molecule of recombinant DNA.

A further purpose of the invention is the use of said IS element, or fragments thereof, for the preparation of hybridization probes useful for the characterization and epidemiological study of strains of C. diphtheriae.

Still further purposes of the invention will become clear fron the hereunder disclosure and examples.

According to the present invention, the element of DNA inserted in the $tox^-$ gene of gamma bacteriophage was cloned and its sequence was analysed by means of known general techniques.

In particular, the lysogen phage of C. diphtheriae C7 ($\gamma$) was induced by adding, to a culture of said strain cultivated at 37°C up to an optical density of 0.4, as measured at 590 nm, mitomycin C at a concentration comprised within the range of from 0.3 to 0.8 mg/ml.

The cellular suspension was then centrifuged and the supranatant, containing the phages, was recovered.

An aliquot of said supranatant, containing from $5 \cdot 10^7$ to $5 \cdot 10^9$ phages was then used for infecting a culture of C. diphtheriae $C7(-)^{tox-}$.

The mixture of lysogen phages and cells of C. diphtheriae C7(-)$^{tox-}$ was kept on a suitable culture medium, at 37°C, up to the complete lysis of the cells.

At the end of the reaction of lysis, the phages were precipitated, by adding polyethyleneglycol to the reaction mixture, and were then purified on a cesium chloride gradient, according to the method as disclosed by Yamamoto ed al (Virology 40, 734-744 (1970)).

The so purified phagic mixture was then kept 10 minutes at 65°C, and to it 0.5 M NaCl was then added; it was then kept at 0°C for a further 15 minutes, for the purpose of extracting the phagic DNA.

The phagic DNA was precipitated with 2.5 volumes of ethanol, it was separated and, after being suspended in a buffer solution (pH 8.0), it was subsequently extracted with phenol and chloroform. At extraction end, the phagic DNA was precipitated again and, after being separated, it was suspended again in phosphate buffer (pH 8.0).

The so-obtained phagic DNA was digested with the restriction enzyme BamHI, according to the modalities as suggested by the manufacturer, and the whole digestion mixture was charged to 1.3%-agarose gel and run at 100 V.

The BamHI fragment, of 5,400 base-pairs, containing the structural gene of the toxin and the insertion element was then electroeluted.

Said BamHI fragment was then digested with HindIII enzyme, and, partially, with ClaI enzyme.

The whole digestion mixture was then charged to 1%-agarose gel and the HindIII-ClaI fragment, of about 2,500 base-pairs, was electroeluted.

Said fragment results to contain the whole sequence of the insertion element, and the 5' terminal portion of the tox gene, up to ClaI site.

As the nucleotidic sequence of the tox gene is known, it was possible to deduce the dimensions of the insertion element, which results to be formed by 1,500 base-pairs (bp).

According to the present invention, the map of restriction of the 1,500-bp element (Figures 1 and 2) and its nucleotidic sequence (Figure 3A) was then determined.

The analysis shows that the sequences at both ends form a 40-nucleotide long repeated inverted imperfect sequence (Figure 3B).

It was furthermore observed that 9 base-pairs of the tox gene, immediately preceding the insertion, are repeated at the end thereof, and that, inside the repeated inverted sequences, a potential gene exists, which could codify for a protein involved in the transposition mechanism.

The dimension of said element, the structure of the end portions, and the presence of said gene are all in accordance with the expected characteristics for the IS insertion elements of the bacteria.

It can be concluded therefore that the element of DNA inserted into the tox gene of the gamma bacteriophage is an IS insertion element.

Said element was given the identification code IScd1, and the fragment of 2,500 bp containing the IScd 1 was amplified and purified by cloning to a suitable vector, from which it can be obtained back by digestion with suitable restriction enzymes.

Cloning vectors suitable for that purpose can be selected from those used in the art. In particular, the pEMBL.8 plasmide of E. coli, described by Dente et al. (Nucleic Acids Research 11, 1645-1655 (1983)) was used.

According to the invention, the plasmidic DNA was truncated with the restriction enzymes HindIII and AccI and was subsequently linked to the HindIII-ClaI DNA fragment of 2,500 bp, in the presence of $T_4$-DNA-ligase, by operating according to known general techniques. The whole ligase mixture was then used for transforming competent cells of E. coli JM 101 (BRL) and the transformed cells were selected by ampicillin-resistance, on LB-containing slabs (DIFCO) to which ampicillin (100 $\gamma$/ml) has been added.

From one of the positive clones, the molecule of recombinant DNA, constituted by pEMBL-8 and by the 2,500-bp HindIII-ClaI fragment was then isolated (Figure 2).

Said molecule was given the identification code pD$\gamma$2, and the strain of E-coli JM 101 transformed with said molecule was deposited with the American Type Culture Center on February 26th, 1986, with the code ATCC 67011.

According to the present invention, the presence of IScd1 insertion elements in the chromosomal DNA of strains of C. diphtheriae was then determined by Southern-blot hybridization.

For that purpose, for the preparation of hybridization probes the whole IScd1 element of 1,500 bp, or its restriction fragments labeled according to one of the known general techniques can be used.

According to a form of practical embodiment of the present invention, the restriction fragment D-SalI, of 600 bp, labeled by nick-translation, was used.

In particular, the chromosomal DNA of strains of C. diphtheriae was digested with different restriction enzymes, and the so-obtained fragments were hybridized with the D-SalI probe in hybridization solution according to the Southern-blot technique (J. Mol. Biol. 98, 503-517, 1975).

The results show a different number of hybridization bands for the different strains. In particular, for C. diphtheriae belfanti 1030, from 15 to 25 hybridization bands were identified, whilst for C. diphtheriae C7(-)$^{tox-}$ only two hybridization bands were identified.

For the purpose of verifying these results, a genomic library generated by starting from C. diphtheriae belfanti 1030 strain was analysed, by slab-hybridization for its sequences homologous to D-SalI fragment.

The positive clones, i.e., those clones which give rise to the hybridization with D-SalI, were subsequently mapped with the restriction enzymes: BglI, BamHI, HindIII, EcoRI, and SalI, and it was thus found that 90% of them contained a DNA segment of about 1,500 bp, the restriction map of which was identical to that of IScd1.

It was furthermore observed that, whilst the lengths of the restriction fragments B, C and D were the same in all clones, those of A and E ranged respectively from a minimum of 500-700 up to a maximum of 9,000-10,000 bp in the different clones.

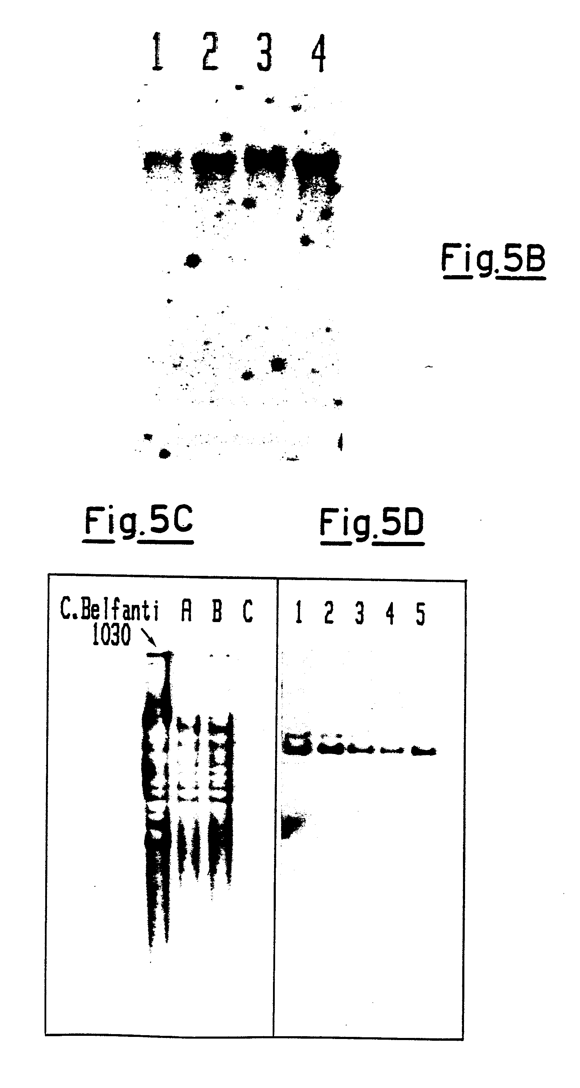

According to the present invention, the hybridization probe obtained from the restriction fragment D-SalI, labeled by nick-translation, was used for an epidemiological study on strains of C. diphtheriae (Table 1) previously studied by classic bacteriologic methods, or by the mapping of the chromosomal DNA, with results being obtained, which not only coincide with the published results, but are absolutely inequivocal, and are simpler to be obtained. For the strains coming from Sweden (Figure 5A), it was demonstrated that all of the isolates classified as mitis, were the same strain. For the isolates coming from Indonesia (Figure 5B), it was demonstrated that the hybridization modalities for the tetracycline-sensitive and tetracycline-resistant strains were the same, thus indicating that the tetracycline resistance is a character recently acquired by tetracycline-sensitive strains of C. diphtheriae strains, which are still now endemic in that area.

Furthermore, the presence of said IScdI element was observed, by means of the chromosomal DNA analysis, in strains isolated in such Countries as U.S.A., Canada, Sweden, Austria and Indonesia, and it was found that the chromosomal distribution of IScdI is identical in strains isolated in the same locality and during the same time period, but is different in the same strains coming from different localities, and isolated during different time periods.

These results indicate that the IScd1 element is present in most strains of C. diphtheriae and that the events of transposition of said element are strain-specific.

Hence, IScd1 or restriction fragments thereof can be used for preparing hybridization probes useful in the diagnostic field and in the epidemiological study of strains of C. diphtheriae.

Brief Description of Figures.

Figure 1: The restriction map of Iscd1 DNA element is shown. The dimensions of the restriction fragments are the following: A = 300 bp; B = 270 bp; C = 180 bp; D = 600 bp; E = 150 bp.

Figure 2: Shows the restriction mapping of the hybrid plasmid pD$\gamma$2.

Figure 3A: Shows the nucleotidic sequence of IScd1 insertion element, inserted in the gene of the diphtherial toxine of gamma bacteriophage. The lower-case letter sequence belongs to the gene of the diphtherial toxine wherein the IScd1 element is inserted. The GGATAGGGG sequence repeated at both IScd1 ends id underlined.

Figure 3B: The nucleotidic sequence is reported of the terminals of IScd1 element, which shows how they are constituted by inverted and repeated sequences which can form the secondary structures reported in Figure.

The lower-case-letter sequences belong to the gene of the diphtherial toxine wherein the IScd1 element is inserted. The underlined sequences are, on the contrary, those repeated at the beginning and at the end of the insertion element.

Figure 4: Southern blot of chrosomomal DNA of C. diphtheriae belfanti 1030 truncated with different restriction enzymes and hybridized with the D-SalI restriction fragment of 600 bp.

Figure 5: Southern-blot of the chromosomal DNA obtained from different isolates of C. diphtheriae by digestion with BamHI. The D-SalI fragment is used as the hybridization probe.

Figure 5A: Strains Isolated in Sweden The lines 1 through 4 contain the DNA respectively obtained from C. diphtheriae mitis CCUG (Culture Collection, University of Gotheborg, Department of Clinical Bacteriology, Guldresgaren, 10, S-413 Gotheborg) 15935, 16574, 17903, and 17269 strains, lines 5 and 6 contain the DNA from C. diphtheriae gravisCCUG 17274 and 17141 strains.

Figure 5B: Strains of C. diphtheriae Isolated in Indonesia (furnished by U.S. Naval Medical Research Unit, APO San Francisco) Lines 1 and 2 contain the DNA of tetracycline-resistant 105 and 126 strains, whilst lines 3 and 4 contain the DNA from tetracycline-sensitive strains 227 and 402.

Figure 5C: Strains of C. diphtheriae A, B and C Isolated at Toronto (coming from The Biological Laboratories Harward University, Boston) The first line contains the chromosomal DNA of C. diphtheriae belfanti 1030. Most of bands of A and B strains co-migrate with those of C. diphtheriae belfanti 1030.

Figure 5D: Strains if C. diphtheriae isolated at Manchester (coming from the Biological Laboratories Harward University, Boston)

The following experimental Examples are illustrative and not limitative of the same invention.

Example 1

Cloning and Sequence Analysis of DNA Element Inserted in the Gene of Toxin of Gamma Bacteriophage

Cells of C. diphtheriae C7 (γ) (described in Buck G. et al, 1981, J. Bacterial, 148, 143-152) are cultivated overnight at 37°C overnight in 100 ml of PTY medium, the composition of which is as follows:

10 g of casamino acids (Difco Laboratories, Detroit, Mich)
10 ml of 1.0% L-tryptophan
2.0 ml of Solution II
1.0 ml of Solution III
0.5 ml of 0.18% calcium pantothenate
1 l of water.

The composition of Solution II and Solution III is reported in Table 2.

The culture medium is sterilized at 115°C and for 15 minutes and, before use, to it 3.0 ml of a sterile 50%-maltose, 0.5%-$CaCl_2$ solution is added.

The culture is then diluted with PTY medium up to an optical density of 0.1 as measured at 590 nm and is then maintained at 37°C up to an optical density of 0.4 as measured at 590 nm.

At the end of said time period, to the culture 0.5 mg/ml of mitomycin C is added and, after 2 hours at room temperature, it is centrifuged and the supranatant, containing $10^6$ phages/ml, is recovered.

One ml of the phagic mixture is used to infect 0.1 ml of culture of C. diphtheriae $C7(-)^{tox-}$, grown up to an optical density of 0.3.

In practice, the process is carried out by blending the phages and the cells of C. diphtheriae $C7(-)^{tox-}$ in 3 ml of PTY top agar, and inoculating the blend onto PTY medium-agar slabs.

After 1 night at 37°C, the phages, which have completely lysed the cells, are collected, separated from agar by centrifuging at 10,000 rpm for 15 minutes, and used for infecting a culture of C. diphtheriae $C7(-)^{tox-}$ in 300 ml of PTY medium at an optical density of 0.25.

The cells are then grown up to an optical density of about 0,8-0,9 and, as soon as the cellular lysis, which can be detected by means of the fast decrease of the optical density, starts, 5 ml of chloroform is added.

The cellular lysis is completed by maintaining the culture at 37°C for 30 minutes.

At the end of said time period, the phages are precipitated with polyethyleneglycol and cesium chloride gradient, as described by Yamamoto.

The phages are then dialyzed against 10 mM Tris buffer, 1 mM EDTA (pH 8.0) for removing the residual cesium chloride, and to them sodium dodecyl sulphate (SDS) is added, to an end concentration of 0.5%.

The so-obtained mixture is maintained at 65°C for 10 minutes, at 0°C for 15 minutes, to it NaCl is added up to an end concentration of 0.5 M, it is kept at 0°C for 15 minutes, and is finally centrifuged. The phagic DNA, after precipitation by means of the addition of an equal volume of isopropanol, is separated from the reaction mixture by centrifugation, is suspended in 0.5 ml of 10 mM Tris, 1mM EDTA (pH 8.0), and is extracted twice with 0.5 ml of phenol and twice with 0.5 ml of chloroform.

At extraction end, the phagic DNA is precipitated again with isopropanol and, after separation by centrifugation, is re-suspended in 0.5 ml of 10 mM Tris, 1 mM EDTA (pH 8.0).

10 μg of phage DNA is digested with 10 units (U) of BamHI restriction enzyme, according to the modalities as suggested by the manufacturer (BRL) and the digestion mixture is charged to 1.3%-agarose gel in acetate buffer (50 mM TRIS, 20 mM EDTA, 18 mM NaCl), with a potential difference of 100 V being applied.

The BamHI fragment, of 5,400 base-pairs, containing the toxin gene and the insertion element, is electroeluted and subsequently truncated with 10 U of HindIII enzyme, and, partially, with 1 U of ClaI, according to the modalities as suggested by the manufacturer.

The whole digestion mixture is charged to 1%-agarose gel and the HindIII-ClaI fragment, of approximately 2,500 base-pairs, is electroeluted.

Said fragment of 200 bp contains the whole insertion element, and the 5′ end portion of the toxin gene up to ClaI site.

As the nucleotidic sequence of the tox gene, wherein the element is inserted, is known, it is possible to deduct the dimension of this element, which results of about 1,500 bp.

In Figure 2, the restriction map of the 1,500-bp element is reported; the dimensions of its restriction fragments are:

A = 300 bp; B = 270 bp; C = 180 bp; D = 600 bp; E = 150 bp.

The HindIII-ClaI fragment of 2,500 bp is then cloned to pEMBL.8+ plasmid, previously truncated with 1 U of HindIII and 1 U of AccI.

The so-obtained hybrid molecule, denominated as pD$\gamma$2 is then used for transforming cells of E. choli JM 101 (BRL).

By subcloning the pD$\gamma$2 hybrid to single-filament vectors M13 mp8 and mp9, is was possible to identify the nucleotidic sequence of the whole insert (Figure 3).

The results reported in Figure (3A and 3B) show that 9 base-pairs of tox gene immediately preceding the insertion are then repeated at the end of this latter.

The sequences at the two ends of the insertion form an inverted and imperfect repeated sequence extending over 40 nucleotides. Furthermore, between the two inverted repeated sequences a potential gene exists, which could codify for a protein involved in the transposition mechanism.

The IS insertion element so obtained is given the code IScd1.

## Example 2

### Use of the D Fragment of IScd1 Element for the Preparation of the Hybridization Probe

10 g of the pD$\gamma$2 hybrid plasmid is digested with 20 U of the SalI restriction enzyme according to the modalities as suggested by the manufacturer.

The so-digested DNA is then charged to 1.5%-agarose gel and is run at 100 V for 2 hours.

At the end of said time period, on the gel two well-separated bands can be observed: one of about 6,000 bp, and the other one of 600 bp, corresponding to the D-SalI fragment of IScd1 element.

The 600-bp fragment is electroeluted from the gel, and is recovered in a homogeneous form by means of precipitation with ethanol.

Said so-purified fragment is then labeled by nick-translation and is used as a hybridization probe on nitrocellulose filters containing the denatured chromosomal DNA from various strains of C. diphtheriae.

The hybridization is carried out for 20 hours, at 65°C, over 5$^{\times}$ SSC medium (1$^{\times}$ SSC = 0.15 M NaCl, 0.015% M sodium citrate) (pH 7.2).

At the end of the hybridization reaction, the filters are washed 3 times with 50 ml of 2$^{\times}$ SSC at 65°C for a total time of 2 hours, and are then submitted to an autoradiography.

After an exposure time ranging from 18 to 48 hours, the films are developed and reviewed.

## Example 3

### Determination of the IScd1 Element in the Chromosomal DNA of strains of C. Diphtheriae

The C. diphtheriae belfanti 1030 strain (Rappuoli R. et al, J Nirol. 45, 524-530 (1983)) is grown in 100 ml of PTY medium, overnight, at 37°C, and 0.3 ml of said culture is subsequently used for inoculating 10 ml of a previously sterilized PTY medium. The culture is incubated with mild stirring, 200 rpm, at 37°C for 3 hours, and after the addition of 1 µg/ml of penicillin G, is maintained at 37°C for 3 hours.

At the end of said time period the cells are separated from the culture by centrifugation, and are resuspended in 2 ml of 10 mM-Tris, 0.5 M-saccharose and 5 mg/ml-lysozyme solution (pH 8.2).

The cellular suspension is then maintained at 37°C for 10 minutes, is centrifuged, and the so-separated cells are re-suspended in 0.5 ml of 10 mM-Tris, 10 mM-EDTA solution (pH 8.2).

The cells are lysed by adding to that solution 40 µl of 20% SDS at 65°C for 20 minutes.

The cellular suspension is then cooled to 37°C and to it 50 µg/ml is added of a solution containing 50 mg/ml of pronase

The culture is incubated at 37°C overnight and the DNA is then sequentially extracted, once with 0.5 ml of phenol, twice with 0.5 ml of phenol, and twice with 0.5 ml of chloroform.

The chromosomal DNA is precipitated from the reaction mixture by means of the addition of 50 µl of 5 M NaCl and 0.6 ml of isopropanol.

The precipitated DNA is separated by centrifugation at 10,000 rpm for 20 minutes, and is re-suspended in 100 µl of water, to an end concentration of 1 µg/µl.

3 µg of DNA is digested with 5 U of each of the following restriction enzymes: BglI, XhoI, ClaI, EcoRI, HindIII and BamHI and the digestion mixture is charged to 1.3%-agarose gel in Tris-acetate buffer.

After 4 hours at 110 V, the gel is removed, coloured with ethidium bromide and transferred over nitrocellulose, according to the Southern-blot technique.

The filters are then hybridized with a probe of radioactive DNA prepared as reported in Example 2. The results obtained (Figure 4) demonstrate that said probe hybridizes from 15 to 25 fragments derived from the digestion with different restriction enzymes.

The same probe determines an individual SalI fragment very intensely. That indicates that a sequence homologous to fragment D is present in chromosome of C. diphtheriae belfanti 1030 in multiple copies and at different sites.

The same tests carried out with C. diphtheriae C7(-)$^{tox-}$ strain show two bands of hybridization, thus indicating the presence of two IScd1 elements.

In order to explain these facts, a genomic library of C. diphtheriae built in lambda EMBL4 vector (described in Frishauf A. et al. J. Mol. Biol. 170, 827-842 (1973)) is screened by hybridization over slab for identifying the sequences homologous to D-SalI fragment.

On 120 examines slabs, 14 positive clones are found. 12 of these clones, mapped with BglI BamHI, HindII; EcoRI and SalI (Boehringer) restriction enzymes, by operating according to the modalities recommended by the manufacturer, present in their DNA a segment of about 1,500 bp identical to IScd1.

## Example 4

### Use of IScd1 Element as Hybridization Probe for Epidemiological Investigations

The hybridization probe prepared as reported in preceding Example 2 is used for an epidemiological investigation on C. diphtheriae strains (Table 1) previously studied by classic techniques.

The strains are grown in PTY medium by operating as reported in Example 2, and 3 µl of chromosomal DNA is digested, after separation, with 5 U of BamHI restriction enzyme.

The digestion mixture is charged to 1.3%-agarose gel in Tris-acetate buffer and is run at 110 V for 3 hours. At the end, the gel is removed, coloured with ethidium bromide, transferred over nitrocellulose and hybridized with the hybridization probe according to the Southern-blot method.

The results obtained are reported in Figures 5A through 5D.

In particular, the results shown in Figure 5A for the strains isolated in Sweden show that strains indicated as mitis have the same hybridization modalities (lines 1 through 4) and that these are well-distinguishable from those shown by strains identified as gravis (lines 5 and 6).

The strains coming from Indonesia (Figure 5B) show one band of hybridization only, both for tetracycline-sensitive (lines 3 and 4) and for tetracycline-resistant strains (lines 1 and 2).

That indicates that the character of resistance to that antibiotic is a recently acquired character.

In Figure 5C, the results obtained for strains isolated at Toronto (A, B and C) and for C. diphtheriae belfanti 1030 isolated in Austria in 1953 are reported.

A, B and C strains had been described by Chang et al. (J. Clin. Microbiol. 8, 767-768 (1978)) and identified as C. belfanti.

A and C strains were toxinogenic and distinguishable from each other both morphologically and on the basis of their bacteriophage.

B strains were not toxinogenic, and were morphologically indistinguishable from A.

The identity of A and B strains, and their difference from C are shown in Figure 5C.

A and B show indeed an identical modality of hybridization, whilst C does not contain any copies of IScd1 insertion element.

In Figure 5D, finally, the results obtained with toxinogenic and non-toxinogenic strains coming from Manchester are displayed.

Said strains, although show a different toxinogenicity, were indistinguishable from one another, and displayed differences relatively to those isolated during preceding epidemics.

The results obtained and reported in Figure 5D confirm the findings by Pappenheimer et al. (J. Hyg. 93 397-404 (1984)) for said C. diphtheriae strains.

TABLE 1

| Corynebacteria Used | | | Origin |
|---|---|---|---|
| C.d. | belfanti 1030 | tox- | Austria 1953 |
| " | C7 | tox- | U.S.A. 1950 |
| " | PWB var. intermedius | tox+ | U.S.A. 1896 |
| " | A003 var. mitis | tox+ | U.S.A. - |
| " | S-601 var. mitis | tox- | U.S.A. 1979 |
| " | A var. mitis | tox+ | Isolated in an out- |
| " | B var. mitis | tox- | break of diphtheria |
| " | C var. mitis | tox+ | in Toronto, in 1977 |
| " | 1 var. mitis | tox+ | |
| " | 2 var. mitis | tox+ | Isolated in an out- |
| " | 3 var. mitis | tox+ | break of diphtheria |
| " | 4 var. mitis | tox+ | in Manchester, in |
| " | 5 var. mitis | tox+ | 1977 |
| " | CCUG15935 var. mitis | tox+ | |
| " | CCUG16574 var. mitis | tox+ | |
| " | CCUG15935 var. mitis | tox+ | |
| " | CCUG17903 var. mitis | tox+ | Isolated in Sweden |
| " | CCUG17269 var. mitis | tox+ | during 1976-1985 |
| " | CCUG17274 var. gravis | tox- | and collected by |

TABLE 1 (cont.d)

| Corynebacteria Used | | | Origin |
|---|---|---|---|
| " | CCUG17141 var. gravis | tox+ | the Culture Collection, University of Gotheborg |
| " | 105 tet.-res. | tox+ | |
| " | 126 tet.-res. | tox+ | Isolated in Jakarta Indonesia, during 1979, 1980 |
| " | 227 tet.-res. | tox+ | |
| " | 402 tet.-res. | tox+ | |
| C. ulcerans 299G | | | Rumania |
| C. ulcerans 9304 | | | Rumania |

TABLE 2

Solution II

| | | |
|---|---|---|
| $MgSO_4.7H_2O$ | 22.5 g | |
| β-alanine | 115 mg | |
| Nicotinic acid | 115 mg | - Add 1 ml of $H_2O$, then concentrated HCl. When dissolved, add the other components of the solution. |
| Pimelic acid | 7.5 mg | |
| 1% $CuSO_4.5H_2O$ | 5 ml | |
| 1% $ZnSO_4.5H_2O$ | 4 ml | |
| 1% $MnCl_2.4H_2O$ | 1.5 ml | |
| Conc. HCl | 3 ml | |

Water q.s. to 100 ml.

Solution III

| | |
|---|---|
| L-cystine | 20 g |
| Conc. HCl | 20 ml |

TABLE 2 (cont.d)

Solution III (cont.d)

Water q.s. to 100 ml

Maltose-$CaCl_2$-Solution

| | |
|---|---|
| Maltose | 50 g |
| 50% $CaCl_2$ | 2 ml |
| $KH_2PO_4$ | 1 g |

Adjust volume to 100 ml.

Adjust pH at 7.4.

Filter on Whatman 40 paper.

Process in autoclave

## Claims

1. Element of DNA of approximately 1,500 base-pairs endowed with typical properties of an IS insertion element, present in one or more copies in the chromosomal DNA of strains of Corynebacterium diphtheriae, useful for the preparation of strain-specific hybridization probes.

2. Element of DNA according to claim 1, the nucleotidic sequence of which is:

1 ctactggGGA TAGGGGAGAG TGTCCGATTG TTTGTGTGCG GGGGCTTGGT
51 TTACAAGTAG TCCGCGAATC GGTCGGGGTA AGCCACGGCT AGTTGGTTGA
101 TGGCTTGTTT CCACCCGGTG GCTTTCGCTC CTTCAATATA GCCGTTGCAT
151 TCGATGGCGC GCTTCGCTTT CTTCGCTCGC TGGGCAGCGC GCTTGTCCTC
201 GATGTTGCAG ATCATCAGCC ACAGCGTTTT CAGCGCCGCG GTATCGTTCG
251 GGAACTGGCC GCGGTTGCGG GTGGCTTTCC GCAGCTCAGC ATTCAGCGAT
301 TCGATCGAAT TCGTGGTGTA GAGTACCCGC CGTGCCGCAG GCGGGAACTG
351 CAAAAATGGC ACAAATCGAT CCCAGGCGTC GCGCCAGACT TTGACCGATT
401 GCGGGTATTT ACGGCCCAGT TCACTGGCCT CGAATGCGTC CAGGGCGGCG
451 CGGGCGGTGT CCTCGGTCGG TGCGGTGTAG ACCTCCCGTA GCGCACTTGA
501 TACGGGTTTG CGGTCTTGGT AGGACACCCA CCTGTTGGCC GCCCGGATCA
551 GGTGCACGAT ACAGGTCTGC ACCATGGAAT TCGGCCAGGT TGCCTCCACG
601 GCTTCCGGCA GGCCTTTGAG CCCGTCGCAG CAGACGATGA ACGCGTCTTG
651 GACCCCACGG TTGGCAAGGT CAGCGCACAC GGATGCCCAA AAACTGGCGC
701 CTTCATTGTC TGCGATCCAC AATCCCAGGA TGTGCTTGAT GCCGTCCATG
751 TCGACGCCTA CCGCCATGTA GCAAGACTTG TTGACCACGC GGTGGCCATC
801 GCGGATCTTC ACTCGCAGCG CGTCGAGGAA GATCACTGGG TAGAACTCGT
851 CGAGCTGGCG GTTTTGCCAG ATCATGACCT CTTCTAACAC CGCGTCGGTA
901 ATGGTGCTGA TCGTATCTGG GCTCATATCC ATCGAGAGGG TGGTGGCGAG
951 GTGGTGCTGA ATATCGCGCA CGGTCATCCC ACCGGCGTAC AGGGAGATGA
1001 TCATGTCATC AAGCTCCGTC AGACGGCGGG TGCCTTTAGG CAGCATCCGC
1051 GGCGTAAACG TGCCAGCTCG ATCCCGGGGC ATAGTCACAT CAAAGGTGCC
1101 GTAGCCAGAG TTGACGGTTT TCGTGTACGA TTAGTTGCGG TGATTGCCAC
1151 CATCCGGGGT GCCCAGCTGG GCTTTCGCCG TACGATCAGA GTGCGCGTAT
1201 CCTAGATGCG CGTCCATTTC AGCCTGCAGA CCAGCGTTGA TCGATGCCTG
1251 CAAAAGACCT TTGACCAGGT CGCTGGCATC ATCGGTGGAA GTCGACAAAT
1301 CGCCGATCAG TTCGGCGATT TCCGGATTTT CCATCAGCTT AGCGCTGATC
1351 TCGTTGACCC TGTTCGGGTC ATGACCTTTC CTCGGTGACA CGGTAGTCAT
1401 TATCAGTGAA ACTCCTTCTG GATCAGAGCC TCACACACAA AACACCTGAC
1451 ACCCTCggat aggggcccca ccttcagccc atgcaggcgc tgatgatgtt

3. Molecule of recombinant DNA obtained from the splicing of a cloning vector with a fragment of DNA containing the insertion element according to claim 1.

4. Molecule of recombinant DNA according to claim 3, wherein the cloning vector is the plasmid of E. coli pEMBL8.

5. Microorganism transformed with the molecule of recombinant DNA according to claims 3 and 4.

6. Microorganism according to claim 5 E. coli ATCC. 67011.

7. Use of the element of DNA or of fragments of restriction thereof according to claim 1, for the preparation of hybridization probes useful for the characterization and epidemiological study of strains of Corynebacterium diphtheriae.

Fig.1

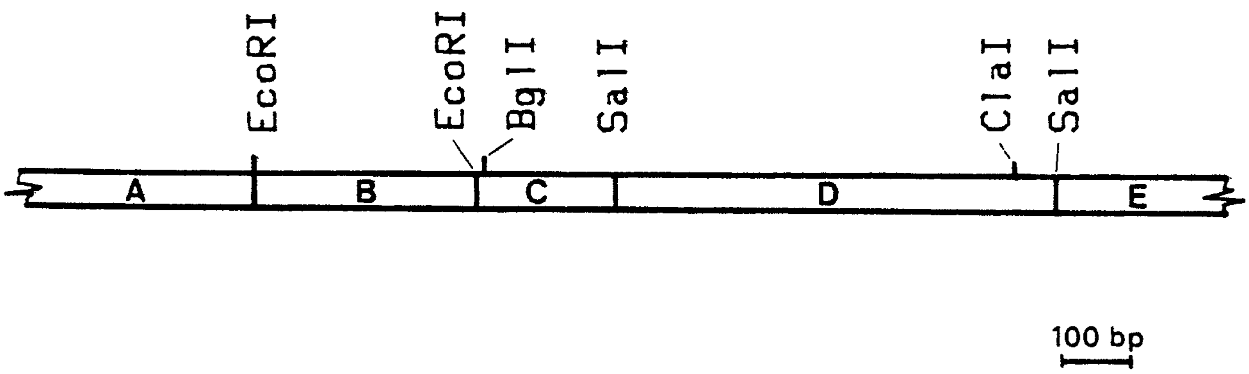
EcoRI
EcoRI
BglI
SalI
ClaI
SalI
A
B
C
D
E
100 bp

Fig.2

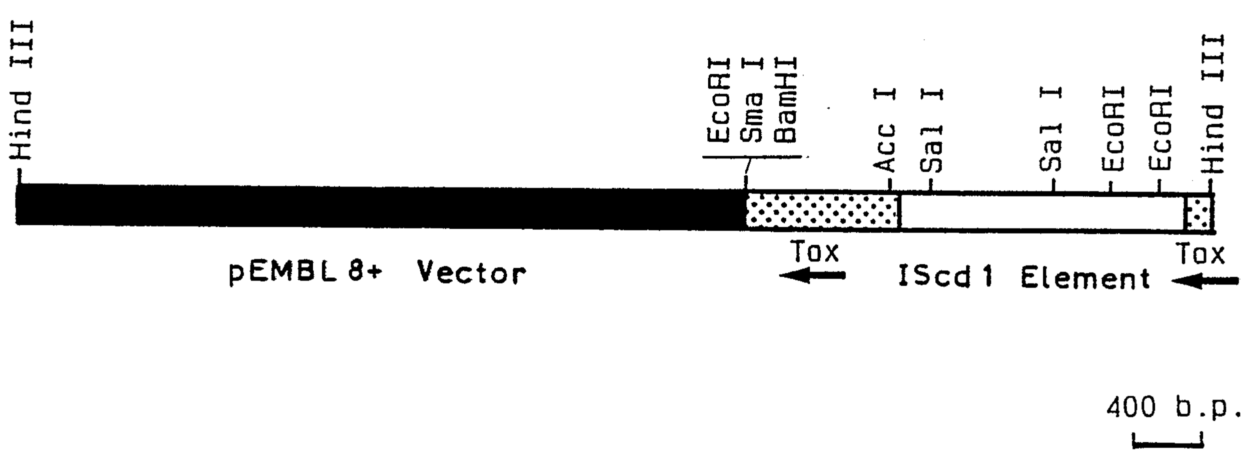
Hind III
EcoRI
Sma I
BamHI
Acc I
Sal I
Sal I
EcoRI
EcoRI
Hind III
pEMBL 8+ Vector
Tox
IScd 1 Element
Tox
400 b.p.

# Fig.3A

```
   1 ctactggGGA TAGGGGAGAG TGTCCGATTG TTTGTGTGCG GGGGCTTGGT
  51 TTACAAGTAG TCCGCGAATC GGTCGGGGTA AGCCACGGCT AGTTGGTTGA
 101 TGGCTTGTTT CCACCCGGTG GCTTTCGCTC CTTCAATATA GCCGTTGCAT
 151 TCGATGGCGC GCTTCGCTTT CTTCGCTCGC TGGGCAGCGC GCTTGTCCTC
 201 GATGTTGCAG ATCATCAGCC ACAGCGTTTT CAGCGCCGCG GTATCGTTCG
 251 GGAACTGGCC GCGGTTGCGG GTGGCTTTCC GCAGCTCAGC ATTCAGCGAT
 301 TCGATCGAAT TCGTGGTGTA GAGTACCCGC CGTGCCGCAG GCGGGAACTG
 351 CAAAAATGGC ACAAATCGAT CCCAGGCGTC GCGCCAGACT TTGACCGATT
 401 GCGGGTATTT ACGGCCCAGT TCACTGGCCT CGAATGCGTC CAGGGCGGCG
 451 CGGGCGGTGT CCTCGGTCGG TGCGGTGTAG ACCTCCCGTA GCGCACTTGA
 501 TACGGGTTTG CGGTCTTGGT AGGACACCCA CCTGTTGGCC GCCCGGATCA
 551 GGTGCACGAT ACAGGTCTGC ACCATGGAAT TCGGCCAGGT TGCCTCCACG
 601 GCTTCCGGCA GGCCTTTGAG CCCGTCGCAG CAGACGATGA ACGCGTCTTG
 651 GACCCCACGG TTGGCAAGGT CAGCGCACAC GGATGCCCAA AAACTGGCGC
 701 CTTCATTGTC TGCGATCCAC AATCCCAGGA TGTGCTTGAT GCCGTCCATG
 751 TCGACGCCTA CCGCCATGTA GCAAGACTTG TTGACCACGC GGTGGCCATC
 801 GCGGATCTTC ACTCGCAGCG CGTCGAGGAA GATCACTGGG TAGAACTCGT
 851 CGAGCTGGCG GTTTTGCCAG ATCATGACCT CTTCTAACAC CGCGTCGGTA
 901 ATGGTGCTGA TCGTATCTGG GCTCATATCC ATCGAGAGGG TGGTGGCGAG
 951 GTGGTGCTGA ATATCGCGCA CGGTCATCCC ACCGGCGTAC AGGGAGATGA
1001 TCATGTCATC AAGCTCCGTC AGACGGCGGG TGCCTTTAGG CAGCATCCGC
1051 GGCGTAAACG TGCCAGCTCG ATCCCGGGGC ATAGTCACAT CAAAGGTGCC
1101 GTAGCCAGAG TTGACGGTTT TGGTGTACGA TTAGTTGCGG TGATTGCCAC
1151 CATCCGGGGT GCCCAGCTGG GCTTTCGCCG TACGATCAGA GTGCGCGTAT
1201 CCTAGATGCG CGTCCATTTC AGCCTGCAGA CCAGCGTTGA TCGATGCCTG
1251 CAAAAGACCT TTGACCAGGT CGCTGGCATC ATCGGTGGAA GTCGACAAAT
1301 CGCCGATCAG TTCGGCGATT TCCGGATTTT CCATCAGCTT AGCGCTGATC
1351 TCGTTGACCC TGTTCGGGTC ATGACCTTTC CTCGGTGACA CGGTAGTCAT
1401 TATCAGTGAA ACTCCTTCTG GATCAGAGCC TCACACACAA AACACCTGAC
1451 ACCCTCggat aggggcccca ccttcagccc atgcaggcgc tgatgatgtt
```

# Fig.3B

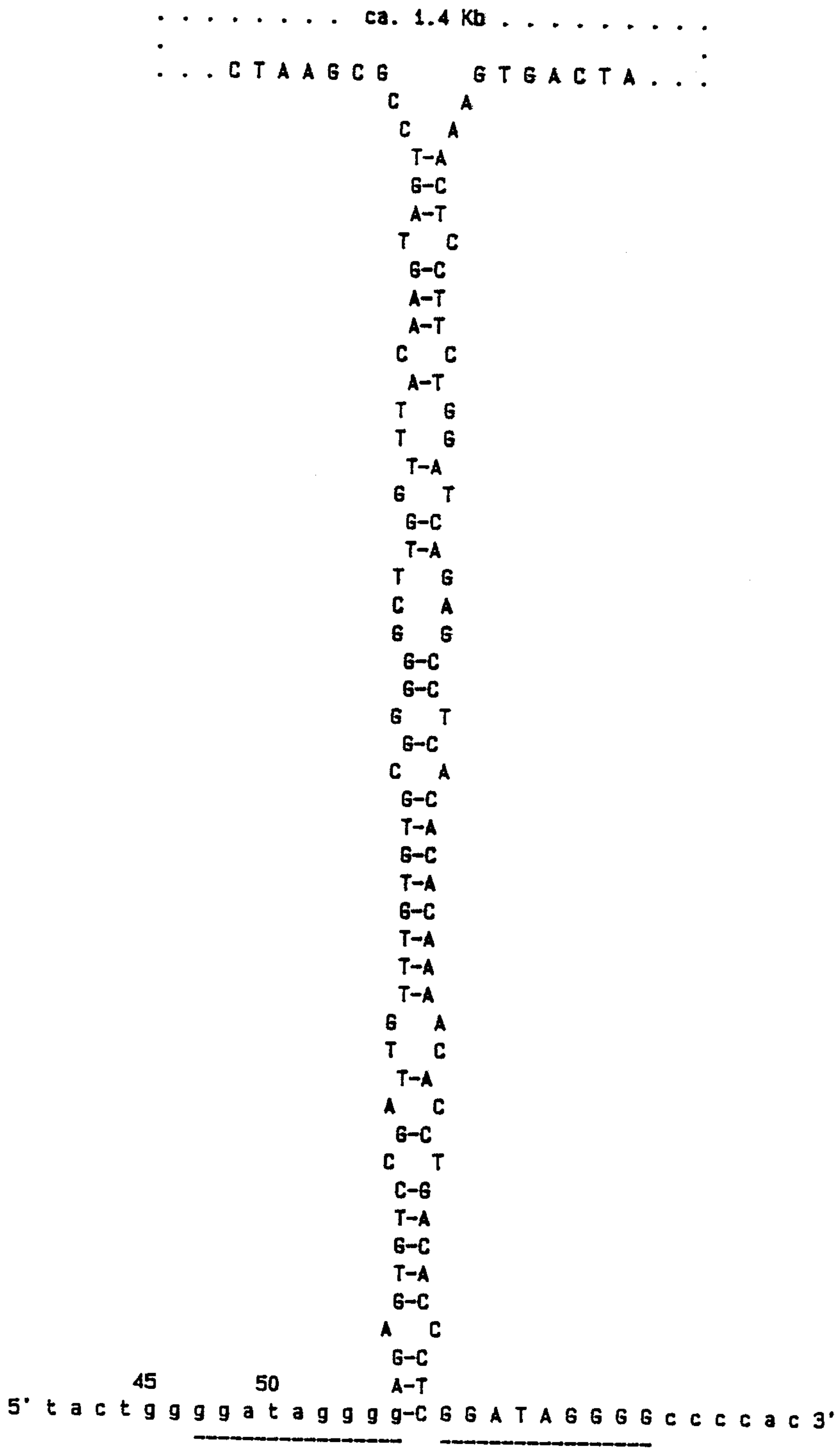
ca. 1.4 Kb
...CTAAGCG GTGACTA...
C A
C A
T-A
G-C
A-T
T C
G-C
A-T
A-T
C C
A-T
T G
T G
T-A
G T
G-C
T-A
T G
C A
G G
G-C
G-C
G T
G-C
C A
G-C
T-A
G-C
T-A
G-C
T-A
T-A
T-A
G A
T C
T-A
A C
G-C
C T
C-G
T-A
G-C
T-A
G-C
A C
G-C
45
50
A-T
5' t a c t g g g g a t a g g g g-C G G A T A G G G G c c c c a c 3'

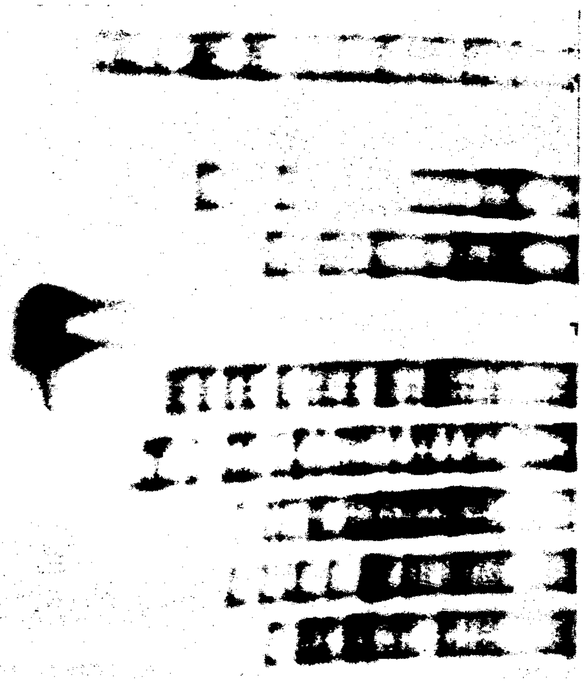

BglI
Sau3a
XhoI
XbaI
SalI
ClaI
EcoRI
BamHI/HindIII
HindIII
BamHI

Fig.4

1 2 3 4 5 6

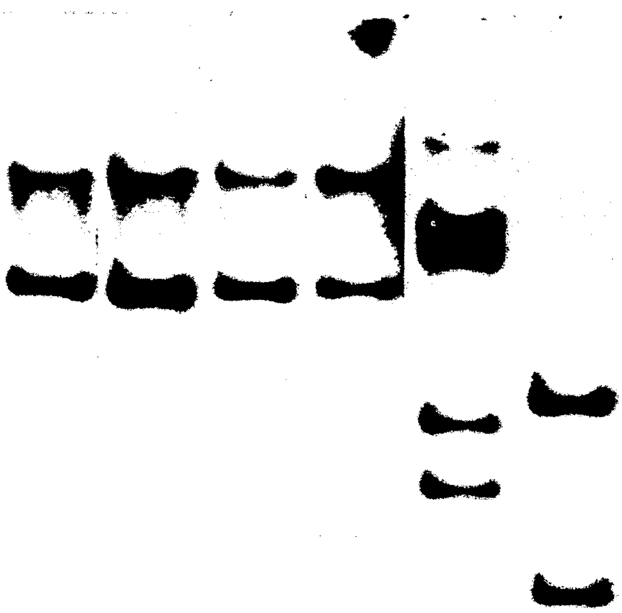

Fig.5A

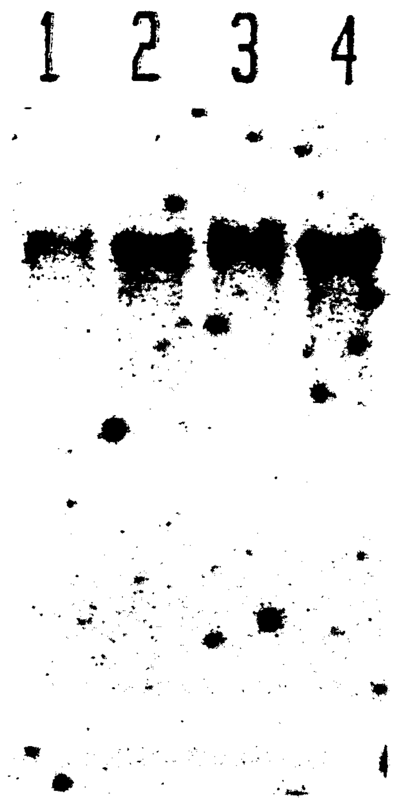


Fig.5B

Fig.5C

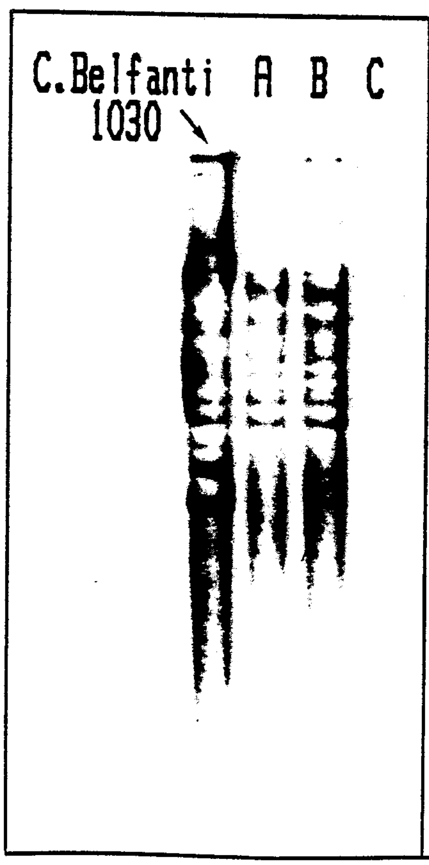


Fig.5D